# EUROPÄISCHE PATENTSCHRIFT

(11) **EP 1 933 843 B1**
(45) Veröffentlichungstag und Bekanntmachung des Hinweises auf die Patenterteilung: **02.02.2011**
(21) Anmeldenummer: 06806225.6
(22) Anmeldetag: 12.10.2006
(51) Int. Cl.: A61K 31/565, A61K 31/57, A61P 15/00, A61P 15/18

(54) **VERWENDUNG VON ESTRADIOLVALERAT ODER ESTRADIOL IN KOMBINATION MIT DIENOGEST ZUR ORALEN THERAPIE DER DYSFUNKTIONELLEN UTERINEN BLUTUNG IN EINHEIT MIT EINER ORALEN KONTRAZEPTION**
USE OF ESTRADIOL VALERATE OR ESTRADIOL COMBINED WITH DIENOGEST FOR THE ORAL THERAPY OF DYSFUNCTIONAL UTERINE BLEEDING IN THE FORM OF ORAL CONTRACEPTIVES
UTILISATION DE VALERATE D'ESTRADIOL OU D'ESTRADIOL COMBINE A DU DIENOGEST POUR TRAITER PAR VOIE ORALE LA MENOMETRORRAGIE SOUS FORME UNITAIRE AVEC UN CONTRACEPTIF ORAL

(30) Priorität: 13.10.2005 EP 05022324
(43) Veröffentlichungstag der Anmeldung: 25.06.2008
(60) Teilanmeldung: 10165860.7
(73) Patentinhaber: Bayer Schering Pharma Aktiengesellschaft, 13353 Berlin (DE)
(72) Erfinder: ZEUN, Susan, 10245 Berlin (DE); BOUDES, Pol, Hackettstown, NJ 07840 (US); ENDRIKAT, Jan, 13125 Berlin (DE); SECCI, Angelo, Parisppany, NJ 07054 (US); ZIMMERMANN, Holger, 14612 Falkensee (DE)
(74) Vertreter: Skødt, Henrik
(86) Internationale Anmeldenummer: PCT/EP2006/009867
(87) Internationale Veröffentlichungsnummer: WO 2007/042296

(56) Entgegenhaltungen:
- EP-A- 0 696 454
- EP-A- 0 770 388
- EP-A1- 1 462 106
- WO-A-2005/102247
- GRASER T ET AL: "Continuous-combined treatment of the menopause with combinations of oestradiol valerate and dienogest: A dose-ranging study" MATURITAS, Bd. 35, Nr. 3, 30. Juni 2000 (2000-06-30), Seiten 253-261, XP002369505 ISSN: 0378-5122
- VON SCHOULTZ B: "Clinical efficacy and safety of combined estradiol valerate and dienogest: a new no-bleed treatment." CLIMACTERIC : THE JOURNAL OF THE INTERNATIONAL MENOPAUSE SOCIETY. AUG 2003, Bd. 6 Suppl 2, August 2003 (2003-08), Seiten 24-32, XP009062446 ISSN: 1369-7137
- DAVIS A ET AL: "Triphasic Norgestimate-Ethinyl Estradiol for Treating Dysfunctional Uterine Bleeding" OBSTETRICS AND GYNECOLOGY, NEW YORK, NY, US, Bd. 96, Nr. 6, Dezember 2000 (2000-12), Seiten 913-920, XP002317447 ISSN: 0029-7844 in der Anmeldung erwähnt

## Beschreibung

### Technisches Gebiet

Die Erfindung betrifft die Verwendung von Estradiolvalerat in Kombination mit 17α-Cyanomethyl-17-β-hydroxyestra-4,9-dien-3on (Dienogest), dabei enthaltend eine erste Phase aus 2 Tagesdosiseinheiten des Estradiolvalerat zu 3 mg eine zweite Phase aus 2 Gruppen von Tagesdosiseinheiten , wobei die erste Gruppe 5 Tagesdosiseinheiten einer Kombination von 2 mg Estradiolvalerat und 2 mg Dienogest und die zweite Gruppe 17 Tagesdosiseinheiten einer Kombination von 2 mg Estradiolvalerat und 3 mg Dienogest enthält,eine dritte Phase aus 2 Tagesdosiseinheiten mit 1 mg Estradiolvalerat und eine weitere Phase aus 2 Tagesdosiseinheiten an pharmazeutisch unbedenklichem Placebo zur Herstellung eines mehrphasigen Kombinationspräparates mit einer Gesamtzahl von 28 Tagesdosiseinheiten zur oralen Therapie der dysfunktionellen uterinen Blutung in Einheit mit einer oralen Kontrazeption.

Die gesamten Tagesdosiseinheiten der mehrphasigen Kombination und des pharmazeutisch unbedenklichem Placebo entsprechen 28 Tagen.

### Stand der Technik

Die dysfunktionelle uterine Blutung (DUB) ist ein häufiges klinisches Problem in der Gynäkologie und betrifft bis zu 33 % der ambulanten gynäkologischen Arztbesuche (Awwad JT, Toth TL, Schiff I. Abnormal uterine bleeding in the perimenopause. Int J Fertil 1993;38:261-9).
DUB Symptome sind:
- verlängerte Mensturationsblutung (> 7 Tage)
- häufige Blutung (Intervall zwischen den Blutungen von kleiner oder gleich 21 Tage)
- verstärkte Blutung (größer oder gleich 80 ml).
Bei DUB handelt es sich um eine Ausschlussdiagnose, d.h. organische Ursachen wie Myome, Polypen oder Krebs sind vor Stellung dieser Diagnose auszuschließen.

DUB tritt sowohl in Verbindung mit einer Anovulation, als auch mit einer Ovulation auf. Grundlegend für derartige Blutungsstörung ist eine Imbalance zwischen der östrogen-stimulierten Aufbauphase (Proliferation) des Endometrium und der gestagenen Umwandlung des Endometriums. Resultieren die DUB Symptome aus einer chronischen Anovulation ist das Endometrium häufig einer gesteigerten östrogenen Proliferation ausgesetzt. Diese kann, neben den benannten Blutungsstörungen zur Hyperplasie des Endometriums führen. (Speroff et all, Clinical Gynecologic Endocrinology and Infertility, Sixth Edition. Lippincott Williams& Wilkins, 1999).

Die Hyperplasie des Endometriums ist ein Risikofaktor für die Entstehung von Endometriumskrebs.

Fraser, I. S., Aust.NZ J Obstet Gynaecol (1990) 30(4),353-356 offenbart die Behandlung der dysfunktionellen uterinen Blutung mit einer Gabe von 5 mg Norethisteron, 3mal täglich oder 10 mg Medroxyprogesteronacetateines 3mal täglich als alleiniges hoch dosiertes Gestagen jeweils für 14 Tage vom 12. bis 25. Zyklustag in 6 anovulatorischen Frauen und über 20 Tage vom 5. bis 25. Zyklustag in 10 ovulatorischen Frauen. In beiden Gruppen konnte eine Verringerung der Blutungsdauer erreicht werden. Eine zuverlässige Kontrazeption war nicht gegeben.

Hickey M, Higham J und Fraser IS The Chochrane Libary, Issue 3 2004 (Hickey M, Higham J, Fraser IS. Progestogens versus oestrogens and progestogens for irregular uterine bleeding associated with anovulation (Cochrane Review). In: The Cochrane Library, Issue 3, 2004. Chichester, UK: John Wiley & Sons, Ltd) beschreiben in einem Review Artikel die niedrige Toleranz der Frauen gegenüber irregulären und starken Blutungen. Sie beschreiben die Rationale der Anwendung von Gestagenen um eine Umwandlung des Endometriums zu erreichen und somit stabilere Menstruationszyklen zu schaffen. Schlussfolgerung des Artikel ist, das derzeit klinische Daten aus randomisierten Studien fehlen um die Wirksamkeit der beschriebenen Behandlungsmöglichkeiten nachzuweisen.

Steiner, R, und Fink, D Praxis (2002) 91(46), 1967-1974 zeigt ebenfalls auf, dass die Behandlung der dysfunktionellen uterinen Blutung u.a. mit hoch dosierten Gestagenen, Estrogenen oder einer Kombinationen von Beiden erfolgen sollte.
Ein Anwendungsregime sieht Steiner in der oralen Gabe von 0,01 mg Ethinylestradiol mit 2 mg Norethisteronacetat für 8 Tage in absteigender Dosierung, nämlich 6,5,4,3,3,3,3,3/Tag. Neben der hormonellen Beeinflussung postuliert Steiner die Möglichkeit der Behandlung in einer akuten Blutungssituation mit Tranexamsäure bis 4x2 Tabletten pro Tag.

Davis A, Obstet gynecol. (2000) 96(6), 913-920 zeigt die Behandlung von dysfunktionellen uterinen Blutungen mittels einer dreistufigen Gabe von Ethinylestradiol (EE)/Norgestimate (NGM) gefolgt von einer hormonfreien Placebo-Gabe für drei 28-Tage Zyklen auf. Das Behandlungsregime gliedert sich dabei derart, dass die Dosis an EE über 21 Tage konstant bleibt (0,035 mg EE), die Dosis an NGM über 21 Tage ansteigt (7 Tagesdosiseinheiten zu 0,180 mg NMG und 7 Tagesdosiseinheiten zu 0, 215 mg NMG und 7 Tagesdosiseinheiten zu 0, 250 mg NMG), gefolgt von einer 7tägigen hormonfreien Placebo-Gabe. In der von Davis durchgeführte placebo-kontrollierten Studie wurden 45% Frauen mit verstärkter Menstruationsblutung (Metrorrhagie, Menometrorrhagie und Polymenorrhoe) und circa 55% Frauen mit verminderter Menstruationsblutung (Oligomenorrhoe) eingeschlossen. Die höchste Erfolgsquote im Vergleich zu Placebo konnte in Frauen mit einer verminderten Menstruationsblutung erreicht werden, hier wurden regelmäßige Abbruchblutungen induziert. Die Oligomenorrhoe ist nicht notwendigerweise Bestandteil der Symptomengruppe DUB und als behandlungswürdige Erkrankung anerkannt.

US 6,797,282 erklärt in der Beschreibung allgemein, dass Langzeitanwendungen (3 Monate) oraler Kontrazeptiva zur Behandlung von Menorrhagien - eine Form von dysfunktioneller uteriner Blutung angewendet werden können.

### Darstellung der Erfindung

Der Erfindung liegt die Aufgabe zugrunde, Mittel zur Behandlung von dysfunktionellen uterinen Blutungen zu entwickeln, die generell die Menge der Blutung verringern, und dass Wiederauftreten einer dysfunktionellen Blutung verhindern und gleichzeitig eine zuverlässige, sichere und gut verträgliche orale Kontrazeption sichern.

Unter dem Begriff "dysfunktionelle uterine Blutung" ist hier die verstärkte Blutung, größer oder gleich 80 ml ohne organische Ursache zu verstehen.

Die Aufgabe wird erfindungsgemäß durch die Verwendung von Estradiolvalerat in Kombination mit 17α-Cyanomethyl-17-β-hydroxyestra-4,9-dien-3on (Dienogest) zur Herstellung eines mehrphasigen Kombinationspräparates zur oralen Therapie der dysfunktionellen uterinen Blutung in Einheit mit einer oralen Kontrazeption gelöst. Die Kombination von Estradiolvalerat mit Dienogest umfasst dabei eine erste Phase aus 2 Tagesdosiseinheiten des Estradiolvalerat zu 3 mg, eine zweite Phase aus 2 Gruppen von Tagesdosiseinheiten , wobei die erste Gruppe 5 Tagesdosiseinheiten einer Kombination von 2 mg Estradiolvalerat , und 2 mg Dienogest und die zweite Gruppe 17 Tagesdosiseinheiten einer Kombination von 2 mg Estradiolvalerat, und 3 mg Dienogest enthält, eine dritte Phase aus 2 Tagesdosiseinheiten mit 1 mg Estradiolvalerat und eine weitere Phase aus 2 Tagesdosiseinheiten an pharmazeutisch unbedenklichem Placebo.
Die gesamten Tagesdosiseinheiten der mehrphasigen Kombination und des pharmazeutisch unbedenklichem Placebo entsprechen 28 Tagen.

Die Dauer der Anwendung umfasst mindestens einen Einnahmezyklus und ist vom individuellen Wunsch der Frau nach Kontrazeption abhängig.

### Untersuchungen zur Wirksamkeit der beanspruchten Formulierung

In einer randomisierten, doppelt-blinden, placebo-kontrollierten, klinischen Studie werden 180 Frauen mit DUB Symptomen und nach Ausschluss einer organischen Ursache der Symptome mit geeigneten diagnostischen Methoden (transvaginaler Ultraschall, Bestimmung von Hormonen im Blut) im Alter zwischen 18 und 50 Jahren, die ihr schriftliches Einverständnis zur Studienteilnahme gegeben haben, behandelt. 120 Frauen erhalten Estradiolvalerat und Dienogest entsprechend der beanspruchten Kombination und 60 Frauen ein Placebo.
Der Versuch umfasst eine Run-in Phase von 90 Tagen in denen die Schwere der Blutungsstörungen aufgenommen werden, 6 Behandlungszyklen und einen Nachbehandlungszyklus (Follow-up-Phase).

Die Blutungsstärke wird durch die alkaline Hämatinmethode quantitativ erfasst. Hierzu werden die Frauen über die gesamte Studie die benutzte Monatshygiene sammeln und beim Prüfzentrum abgeben. Die Dauer der Blutung und die Dauer der blutungsfreien Intervalle werden über tägliche Dokumentation in einem elektronischen Tagebuch erfasst.

## Patentansprüche

1. Ein mehrphasige Kombinationspräparat mit einer Gesamtzahl von 28 Tagesdosiseinheiten von Estradiolvalerat in Kombination mit 17α-Cyanomethyl-17-β-hydroxyestra-4,9-dien-3on (Dienogest), dabei enthaltend eine erste Phase aus 2 Tagesdosiseinheiten des Estradiolvalerat zu 3 mg,
eine zweite Phase aus 2 Gruppen von Tagesdosiseinheiten , wobei die erste Gruppe 5 Tagesdosiseinheiten einer Kombination von 2 mg Estradiolvalerat und 2 mg Dienogest
und die zweite Gruppe 17 Tagesdosiseinheiten einer Kombination von 2 mg Estradiolvalerat und 3 mg Dienogest enthält,
eine dritte Phase aus 2 Tagesdosiseinheiten mit 1 mg Estradiolvalerat und eine weitere Phase aus 2 Tagesdosiseinheiten an pharmazeutisch unbedenklichem Placebo
zur Anwendung in der oralen Therapie der dysfunktionellen uterinen Blutung, worunter eine verstärkte Menstruationsblutung, bei der der Blutverlust größer oder gleich 80 ml beträgt, ohne organische Ursache zu verstehen ist, in Einheit mit einer oralen Kontrazeption.

## Claims

1. A multi-stage combined preparation with a total of 28 daily doses of oestradiol valerate in combination with 17α-cyanomethyl-17β-hydroxyoestra-4,9-diene-3-one (dienogest), comprising
a first stage, with two daily doses of 3 mg of oestradiol valerate,
a second stage, with two sets of daily doses, where the first set comprises five daily doses of a combination of 2 mg of oestradiol valerate and 2 mg of dienogest, and
the second set comprises 17 daily doses of a combination of 2 mg of oestradiol valerate and 3 mg of dienogest,
a third stage, with two daily doses of 1 mg of oestradiol valerate, and another stage of two daily doses of a pharmaceutically acceptable placebo,
for use in the oral treatment of dysfunctional uterine bleeding, which is to be understood as meaning intensified menstrual bleeding in which the blood loss is not less than 80 ml, without an organic cause, in conjunction with oral contraception.

## Revendications

1. Préparation en combinaison polyphasique, ayant un nombre total de 28 unités posologiques journalières de valérate d'oestradiol en combinaison avec de la 17α-cyanométhyl-17β-hydroxyoestra-4,9-dièn-3-one (Dienogest), contenant
une première phase de 2 unités posologiques journalières de valérate d'oestradiol à 3 mg,
une deuxième phase de 2 groupes d'unités posologiques journalières, le premier groupe contenant 5 unités posologiques journalières d'une combinaison de 2 mg de valérate d'oestradiol et de 2 mg de Dienogest, et le deuxième groupe contenant 17 unités posologiques journalières d'une combinaison de 2 mg de valérate d'oestradiol et de 3 mg de Dienogest,
une troisième phase de 2 unités posologiques journalières à 1 mg de valérate d'oestradiol, et une phase supplémentaire de 2 unités posologiques journalières d'un placébo pharmaceutiquement inoffensif,
pour utilisation en thérapie orale de l'hémorragie utérine dysfonctionnelle, ce par quoi il convient d'entendre un renforcement du flux menstruel, où la perte de sang est supérieure ou égale à 80 ml, sans cause organique, en présence d'une contraception orale.
